Europäisches Patentamt

European Patent Office

Office européen des brevets

(19)

(11) Veröffentlichungsnummer : **0 277 996 B1**

(12) **EUROPÄISCHE PATENTSCHRIFT**

(45) Veröffentlichungstag der Patentschrift :
09.10.91 Patentblatt 91/41

(21) Anmeldenummer : 87905740.4

(22) Anmeldetag : 19.08.87

(86) Internationale Anmeldenummer :
PCT/EP87/00465

(87) Internationale Veröffentlichungsnummer :
WO 88/01161 25.02.88 Gazette 88/05

(51) Int. Cl.$^5$ : **A61K 7/13, D06P 3/08**

(54) **MITTEL ZUM OXIDATIVEN FÄRBEN VON HAAREN, VERFAHREN ZU SEINER HERSTELLUNG UND VERWENDUNG DES MITTELS.**

(30) Priorität : 21.08.86 DE 3628397

(43) Veröffentlichungstag der Anmeldung :
17.08.88 Patentblatt 88/33

(45) Bekanntmachung des Hinweises auf die
Patenterteilung :
09.10.91 Patentblatt 91/41

(84) Benannte Vertragsstaaten :
FR GB NL

(56) Entgegenhaltungen :
EP-A- 0 182 187
DE-A- 2 028 818
FR-A- 650 755
FR-A- 1 121 036

(56) Entgegenhaltungen :
PATENT ABSTRACTS OF JAPAN, vol. 9, no.
112 (c-281)(1835), 16May 1985 & JPA 604115 (
KANEBO K.K.) 10 January 1985, see the whole
abstract
CHEMICAL ABSTRACTS, vol. 92, no. 20, 20
May 1980, (Columbus, Ohio, US), E.A. Simonov
et al.:" Spectral studies of fur dyeing with
oxidation dyes", see page 70, abstract
1652322q, & Kozh.-Obuvn. Prom-st. 1980, 22(2),
45-7

(73) Patentinhaber : GOLDWELL
AKTIENGESELLSCHAFT
Zerninstrasse 10-18
W-6100 Darmstadt 13 (DE)

(72) Erfinder : TENNIGKEIT, Jürgen
Felsbergstra e 51
W-6104 Seeheim 2 (DE)
Erfinder : LORENZ, Herbert
Ober-Ramstädter Stra e 22
W-6101 Gro -Bieberau (DE)

## Beschreibung

Die Erfindung betrifft ein Mittel für die oxidative Färbung von menschlichen oder tierischen Haaren, welches unmittelbar vor der Anwendung durch Vermischen eines wenigstens einen Oxidationsfarbstoff enthaltenden Färbemittels mit einem Oxidationsmittel hergestellt ist und einen Katalysator enthält. Außerdem betrifft die Erfindung ein Verfahren zur Herstellung des Mittels sowie seine Verwendung.

Bis in die jüngste Zeit werden für die dauerhafte Färbung von menschlichen Haaren alkalische Oxidationsfarbstoffe enthaltende Färbemittel in Gel- oder Cremeform verwendet, die unmittelbar vor der Anwendung mit einem sauren Oxidationsmittel, z.B. Wasserstoffperoxid, zum gebrauchsfertigen auf das zu färbende Haar aufzutragende Haarfärbemittel vermischt werden. Die Färbung entsteht hierbei durch Reaktion bestimmter Entwicklersubstanzen mit bestimmten Kupplersubstanzen in Gegenwart eines geeigneten Oxidationsmittels. Das gebrauchsfertige Präparat liegt dabei deutlich im alkalischen Bereich, wodurch die Oberflächen der zu färbenden Haare in eine für das Eindringen der Oxidationsfarbstoffe vorteilhaften Weise aufgeschlossen werden. Andererseits beanspruchen alkalische Präparate die Haare auch, so daß es - insbesondere bei wiederholten Einwirkungen, z.B. bei mehrfachem Nachfärben, - zu Schädigungen des Haars kommen kann. Solche Nachfärbungen sind aber - insbesondere bei hellergefärbtem Haar - erforderlich, um beispielsweise den ungefärbten Haarnachwuchs farblich an die bereits gefärbten Haarlängen anzugleichen, bzw. um die früher gefärbten Haarlängen farblich aufzufrischen, wenn sie im Laufe der Zeit durch äußere Einwirkungen, z.B. Sonnenbestrahlung, häufiges Waschen u.dgl. gegenüber der ursprünglichen Farbe aufgehellt bzw. ausgeblichen sind.

Frühere Versuche, die insbesondere in den Haarspitzen auftretenden schädigenden Wirkungen der alkalischen Haarfärbemittel durch saure Einstellung des aus dem Oxidationsfarbstoff und dem Oxidationsmittel fertig aufbereiteten Präparats zu vermeiden, führten zu einer ungenügenden Anfärbewirkung des Mittels. Erst in jüngster Zeit ist von der Anmelderin ein im gebrauchsfertig aufbereiteten Zustand im schwach sauren Bereich (pH-Wert zwischen 5,9 und 6,9) liegendes Mittel entwickelt worden, welches eine gute Anfärbung der behandelten Haare bewirkt, wobei die bei alkalischen Haarfärbemitteln zu beobachtenden Schädigungen der Haare weitestgehend vermieden sind (WO 87/01033). Erreicht wird dieses überraschende Ergebnis bei diesem Haarfärbemittel durch Zugabe von Mangandioxid zum Haarfärbemittel in geringen Mengen, welches bezüglich der Anfärbewirkung als Katalysator wirkt.

Es ist ein Haarfärbemittel der eingangs erwähnten Art bekannt (Patent Abstracts of Japan, Band 9, Nr. 112 (C 281) (1985) ), welches unmittelbar vor der Anwendung aus zwei Lösungen aufbereitet wird, von denen die erste Lösung 0,01 bis 10 Gew.% eines Oxidtionsfarbstoffs mit einer $NH_2$-Gruppe in der Benzol-Seitenkette wie z.B. Aminodiphenylamin, 10 bis 30 Gew.% einer oberflächenaktiven Substanz (vorzugsweise nichtionisch), 10 bis 30 Gew.% eines niederen Fettalkohols und 0,5 bis 2 Gew.% Ammoniak und Wasser enthält. Die zweite Lösung besteht aus 1 bis 6 Gew.% Wasserstoffperoxid, Wasser und - als Katalysator - 0,025 bis 0,5 Gew.%, vorzugsweise aber 0,05 bis 0,25 Gew.% Alkalimetallchlorid (vorzugsweise NaCl oder KCl). Das Mittel wird also mit Ammoniak alkalisch gemacht. Um dennoch eine möglichst schonende Haarbehandlung zu erreichen, ist eine kurze Behandlungszeit vorgesehen, woraus sich jedoch der Nachteil ergibt, daß das Färbeergebnis bei nur kurzen Zeitschwankungen stark variieren kann. In jedem Fall wird das Haar auch bei kurzer Einwirkungsdauer durch die alkalische Lösung strapaziert. Auf eine sich anschließende gründliche Neutralisierung muß genauestens geachtet werden.

Der Erfindung liegt demgegenüber die Aufgabe zugrunde, ein Mittel zum oxidativen Färben von Haaren sowie ein Verfahren zu seiner Herstellung anzugeben, welches bei noch verbesserter Färbewirkung auch bei mehrfacher Anwendung nicht zu den bei Verwendung von alkalischen Haarfärbemitteln beobachteten Haarschädigungen führt.

Ausgehend von einem Haarfärbemittel der eingangs erwähnten Art wird diese Aufgabe erfindungsgemäß dadurch gelöst, daß das Färbemittel creme- oder gelförmig ist, daß die Mischung aus Färbe- und Oxidationsmittel auf einen pH-Wert zwischen 5,9 und 6,9 eingestellt ist, und daß als Katalysator wenigstens ein Salz aus der Gruppe Kaliumjodid, Lithiumchlorid, Kaliumbichromat, Magnesiumacetat, Kalziumchlorid und Bariumnitrat in einer Konzentration zwischen 0,0004 und 0,001 Gew.%, bezogen auf das gebrauchsfertige Haarfärbemittel enthalten ist. Bei Verwendung der erfindungsgemäß vorgesehenen Katalysatoren in einem Haarfärbemittel mit leicht saurem pH-Wert wurde überraschenderweise gefunden, daß im Vergleich zum Stand der Technik bereits geringste Katalysatorenmengen verwendet werden können, nämlich nur 1 bis 1/4% der in der angegebenen japanischen Veröffentlichung genannten Katalysatormenge. Neben einer beträchtlichen Substanzersparnis führt dies bei dem für die Haarstruktur günstigeren pH-Wert zu einem insgesamt milderen Produkt.

Bei der Herstellung des erfindungsgemäßen Haarfärbemittels wird entweder so verfahren, daß das Alkaliund/oder Erdalkalimetallsalz dem Oxidationsfarbstoff vor der Zugabe des - in der Regel flüssigen - Oxidationsmittels zugemischt wird, oder es kann auch mit oder unmittelbar nach der Vermischung des Oxidationsfarbstoffs mit dem Oxidationsmittel zugegeben werden.

Bei der Anwendung des erfindungsgemäßen Haarfärbemittels zum Färben menschlicher Haare wird praktisch in gleicher Weise wie bisher mit alkalischen Haarfärbemitteln gearbeitet. Unmittelbar vor der Anwendung wird aus wenigstens einem crem- oder gelförmigen, wenigstens einen Oxidationsfarbstoff und zusätzlich das Alkali- oder Erdalkalimetallsalz enthaltende Färbemittel durch Zugabe des Oxidationsmittels das gebrauchsfertige Haarfärbemittel aufbereitet, auf das zu färbende Haar aufgetragen und eine vorgegebene Zeitdauer zur Einwirkung gebracht, worauf das Haarfärbemittel ausgewaschen wird. Es hat sich gezeigt, daß hierbei hervorragend haltbare Färbungen von menschlichem Haar in brillanten Farbtönen ermöglicht werden, sofern nicht dunkleres Haar auf einen helleren Farbton umgefärbt werden soll. Die auf die alkalische Einstellung der bis heute überwiegend verwendeten Haarfärbemittel zurückzuführenden Haarschädigungen werden dabei vollständig vermieden.

Für die aufhellende oder heller ziehende Färbung von dunklerem Haar muß allerdings auf die bekannten alkalischen Haarfärbemittel zurückgegriffen werden. Da die Schädigungen der Haare durch solche alkalischen Haarfärbemittel aber in der Regel erst bei mehrfachem Nachfärben auftreten, können solche Schäden beim Nachfärben vermieden werden, indem zunächst auf die dunkleren nachgewachsenen kopfhautnahen Haarlängen ein an sich bekanntes alkalisch eingestelltes Haarfärbemittel und dann das schwach saure Haarfärbemittel auf die bereits früher gefärbten anschließenden Haarlängen aufgebracht wird, worauf sowohl das alkalische als auch das schwach saure Haarfärbemittel nach hinreichender Einwirkungszeit aus dem behandelten Haar ausgewaschen werden.

Besonders problematisch in bezug auf Haarschädigungen ist eine kombinierte Dauerwell-Haarfärbebehandlung. Wesentlich verringert wird die Gefahr von Haarschaden bei einer Anwendung des erfindungsgemäßen Haarfärbemittels, wobei dann so vorgegangen wird, daß auf das gewaschene und auf Wicklern aufgewickelte Haar zunächst ein Dauerwellpräparat für eine vorgegebene Zeitdauer zur Einwirkung gebracht und dann ausgewaschen wird, worauf das gewickelte Haar mit einem flüssigen oxidierenden Fixiermittel vorfixiert und dann nach dem Abnehmen der Wickler mittels des schwach sauren Haarfärbemittels nachfixiert wird, indem dieses für die zur hinreichenden Einfärbung der Haare erforderliche Zeitdauer auf dem Haar zur Einwirkung gebracht und anschließend ausgewaschen wird. Das Haarfärbemittel ersetzt also das beim Dauerwellen üblicherweise beim Nachfixieren verwendete Fixiermittel, d.h. wird sozusagen in Doppelfunktion eingesetzt, wobei die sonst bei Verwendung von alkalisch eingestellten Haarfärbemitteln zu befürchtenden Schädigungen vermieden werden.

Die vorstehend geschilderten Verwendungsmöglichkeiten des erfindungsgemäßen Haarfärbemittels werden nachstehend anhand einer Reihe von speziellen Beispielen näher ausgeführt. Als Beispiel für die Wirksamkeit von Alkalimetallsalzen werden dabei Rezepturen von unter Zugabe von Kaliumjodid aufbereiteten Haarfärbemitteln und als Beispiel für die Wirksamkeit von Erdalkalimetallsalzen mit Magnesiumacetat aufbereiteten Haarfärbepräparaten beschrieben.

## Beispiel 1a

Oxidationsfärbung

20 ml Färbepaste der unten angegebenen Rezeptur für die Farbe "Haselnußblond" mit einem Zusatz von 0,0004 % Kaliumjodid wurde mit 40 ml einer 2 % $H_2O_2$ enthaltenden Entwicklerlösung vermischt, woraus ein Haarfärbemittel mit einem pH-Wert von 6,8 resultierte. Das Mittel wurde auf ein mittelstarkes Haar mit dem Grundton mittelaschblond aufgetragen und 20 Minuten zur Einwirkung gebracht.

Als Ergebnis wurde eine Umfärbung des Haares zu einem intensiven Haselnußblond-Farbton erhalten.

Versuche ergaben, daß die Einwirkungszeit des Haarfärbemittels je nach gewünschter Farbintensität 5 bis 20 Minuten betragen kann.

Rezeptur der Färbepaste "Haselnußblond":

| | |
|---|---|
| Cetylstearylalkohol | 10,0 g |
| Kokosfettsäuremonoäthanolamid | 2,0 g |
| Stearinsäuremonoäthanolamid | 2,0 g |
| Stearinsäurediäthanolamid | 1,0 g |
| p-Toluylendiaminsulfat | 0,25 g |
| m-Aminophenol | 0,01 g |
| Resorcin | 0,01 g |
| p-Aminophenol | 0,08 g |
| p-Amino-o-kresol | 0,06 g |

| | | |
|---|---|---|
| Pikraminsäure | | 0,05 g |
| Monoäthanolamin | | 0,2 g |
| Ammoniumchlorid | | 0,2 g |
| Natriumlaurylsulfat | | 0,3 g |
| Kaliumjodid | | 0,0004 g |
| Natriumsulfit | | 0,25 g |
| Äthylendiamintetraessigsäure | | 0,2 g |
| Parfum | | 0,2 g |
| Enth. Wasser | auf | 100,00 g |

Beispiel 1b

Oxidationsfärbung

20 ml Färbepaste der unten angegebenen Rezeptur für die Farbe "Kupferblond" mit einem Zusatz von 0,0004 % Kaliumjodid wurde mit 40 ml einer 2 % $H_2 O_2$ enthaltenden Entwicklerlösung vermischt, woraus ein Haarfärbemittel mit einem pH-Wert von 6,8 resultierte.

Das Mittel wurde auf ein mittelstarkes Haar mit dem Grundton mittelaschblond aufgetragen und 20 Minuten zur Einwirkung gebracht.

Als Ergebnis wurde eine Umfärbung des Haars in einen dezenten Kupferton erhalten.

Die Einwirkungszeit des Haarfärbemittels kann je nach gewünschter Farbintensität 5 bis 30 Minuten betragen.

Rezeptur der Färbepaste "Kupferblond":

| | |
|---|---|
| Cetylstearylalkohol | 10,0 g |
| Kokosfettsäuremonoäthanolamid | 2,0 g |
| Stearinsäuremonoäthanolamid | 2,0 g |
| Stearinsäurediäthanolamid | 1,0 g |
| p-Toluylendiaminsulfat | 0,20 g |
| p-Aminophenol | 0,70 g |
| p-Amino-o-kresol | 0,70 g |
| Monoäthanolamin | 0,2 g |
| Ammoniumchlorid | 0,2 g |
| Natriumlaurylsulfat | 0,3 g |
| Kaliumjodid | 0,0004 g |
| Natriumsulfit | 0,12 g |
| Äthylendiamintetraessigsäure | 0,2 g |
| Parfum | 0,2 g |
| Enth. Wasser      auf | 100,00 g |

Beispiel 2

Oxidationsfärbung zum schonenden, sauren Ausgleich der Haarspitzen nach normaler, alkalischer Ansatzfärbung

20 ml Färbepaste der beim Beispiel 1a angegebenen Rezeptur für die Farbe "Haselnußblond" mit einem Zusatz von 0,0004 % Kaliumjodid wurde mit 40 ml einer 2 % $H_2 O_2$ enthaltenden Entwicklerlösung vermischt, woraus ein Haarfärbemittel mit einem pH-Wert von 6,8 resultierte.

Das Mittel wurde auf ein mittelstarkes Haar, welches haselnußblond gefärbt war und dessen Längen und Spitzen ausgeglichen sind und einen Ton von hellaschblond haben, aufgetragen und 10 Minuten zur Einwirkung gebracht.

Als Ergebnis wurde ein dem Ansatz angeglichener Haselnußblond-Farbton der ausgeblichenen Längen und Spitzen erhalten. Die Einwirkungsdauer des Präparats kann wiederum entsprechend den auszugleichenden Farbintensitäten zwischen 5 bis 15 Minuten variiert werden.

4

Beispiel 3a

Oxidationsfärbung in Verbindung mit der Dauerwellung

Auf mittelstarkem Haar, das einen Grundton von dunkelgoldblond hatte und dessen Haarlängen und -spitzen ausgeblichen ware, wurde eine Dauerwellung durchgeführt.

Die Dauerwelle wurde bis einschließlich der ersten Fixierung auf dem Wickler nach Gebrauchsanweisung verarbeitet.

Zur Nachfixierung und zum gleichzeitigen Färben wurde nach dem Entfernen der Wickler 20 ml Färbepaste der unten angegebenen Rezeptur für die Farbe "Zyklamen" mit einem Zusatz von 0,0004 % Kaliumjodid mit 40 ml einer Dauerwell-Fixierung mit einem $H_2 O_2$-Gehalt von 2 % vermischt.

Das hieraus resultierende Haarfärbemittel mit einem pH-Wert von 6,8 wurde nun mittels einer Auftrageflasche bzw. eines Schwämmchens auf das Haar aufgetragen und 10 Minuten zur Einwirkung gebracht. Als Ergebnis wurde zusätzlich zur Dauerwellung eine Umfärbung des Haars in einen rotvioletten (Zyklamen)-Farbton erhalten. Je nach gewünschter Farbintensität kann die Einwirkungsdauer zwischen 5 und 15 Minuten variiert werden.

Rezeptur der Färbepaste "Zyklamen":

| | | |
|---|---|---|
| Cetylstearylalkohol | | 10,0 g |
| Kokosfettsäuremonoäthanolamid | | 2,0 g |
| Stearinsäuremonoäthanolamid | | 2,0 g |
| Stearinsäurediäthanolamid | | 1,0 g |
| p-Toluylendiaminsulfat | | 0,5 g |
| p-Amino-o-kresol | | 0,4 g |
| p-Aminophenol | | 0,1 g |
| Monoäthanolamin | | 0,2 g |
| Ammoniumchlorid | | 0,2 g |
| Natriumlaurylsulfat | | 0,3 g |
| Kaliumjodid | | 0,0004 g |
| Natriumsulfit | | 0,25 g |
| Äthylendiamintetraessigsäure | | 0,2 g |
| Parfum | | 0,2 g |
| Enth. Wasser | auf | 100,00 g |

Beispiel 3b

Oxidationsfärbung in Verbindung mit der Dauerwellung

Auf mittelstarkem Haar, das einen Grundton von dunkelgoldblond hatte und dessen Haarlängen und -spitzen ausgeblichen waren, wurde eine Dauerwellung durchgeführt.

Die Dauerwelle wurde bis einschließlich der ersten Fixierung auf dem Wickler nach Gebrauchsanweisung verarbeitet.

Zur Nachfixierung und zum gleichzeitigen Färben wurde nach dem Entfernen der Wickler 20 ml Färbepaste der unten angegebenen Rezeptur für die Farbe "Mahagoni" mit einem Zusatz von 0,0004 % Kaliumjodid mit 40 ml einer Dauerwell-Fixierung mit einem $H_2 O_2$-Gehalt von 2 % vermischt.

Das hieraus resultierende Haarfärbemittel mit einem pH-Wert von 6,8 wurde nun mittels einer Auftrageflasche bzw. eines Schwämmchens auf das Haar aufgetragen und 10 Minuten zur Einwirkung gebracht. Als Ergebnis wurde zusätzlich zur Dauerwellung eine Umfärbung des Haars in einen kräftigen Rotton erhalten. Je nach gewünschter Farbintensität kann die Einwirkungsdauer zwischen 5 und 15 Minuten varriiert werden.

Rezeptur der Färbepaste "Mahagoni":

| | |
|---|---|
| Cetylstearylalkohol | 10,0 g |
| Kokosfettsäuremonoäthanolamid | 2,0 g |
| Stearinsäuremonoäthanolamid | 2,0 g |
| Stearinsäurediäthanolamid | 1,0 g |
| p-Toluylendiaminsulfat | 0,4 g |

| | | |
|---|---|---|
| p-Aminophenol | | 0,4 g |
| p-Amino-o-kresol | | 0,4 g |
| o-Nitro-p-Phenylendiamin | | 0,4 g |
| Monoäthanolamin | | 0,2 g |
| Ammoniumchlorid | | 0,2 g |
| Natriumlaurylsulfat | | 0,2 g |
| Kaliumjodid | | 0,0004 g |
| Natriumsulfit | | 0,25 g |
| Äthylendiamintetraessigsaüre | | 0,2 g |
| Parfum | | 0,2 g |
| Enth. Wasser | auf | 100,00 g |

Beispiel 4a

Oxidationsfärbung

20 ml Färbepaste der unten angegebenen Rezeptur für die Farbe "Haselnußblond" mit einem Zusatz von 0,0006 % Magnesiumacetat wurde mit 40 ml einer 2 % $H_2O_2$ enthaltenden Entwicklerlösung vermischt, woraus ein Haarfärbemittel mit einem pH-Wert von 6,8 resultierte. Das Mittel wurde auf ein mittelstarkes Haar mit Grundton mittelaschblond aufgetragen und 20 Minuten zur Einwirkung gebracht.

Als Ergebnis wurde eine Umfärbung des Haares zu einem intensiven Haselnußlond-Farbton erhalten.

Entsprechen der gewünschten Farbintensität kann die Einwirkungszeit zwischen 5 bis 20 Minuten liegen.

Rezeptur der Färbepaste "Haselnußblond":

| | | |
|---|---|---|
| Cetylstearylalkohol | | 10,0 g |
| Kokosfettsäuremonoäthanolamid | | 2,0 g |
| Stearinsäuremonoäthanolamid | | 2,0 g |
| Stearinsäurediäthanolamid | | 1,0 g |
| p-Toluylendiaminsulfat | | 0,25 g |
| m-Aminophenol | | 0,01 g |
| Resorcin | | 0,01 g |
| p-Aminophenol | | 0,08 g |
| p-Amino-o-kresol | | 0,06 g |
| Pikraminsäure | | 0,05 g |
| Monoäthanolamin | | 0,2 g |
| Ammoniumchlorid | | 0,2 g |
| Natriumlaurylsulfat | | 0,3 g |
| Magnesiumacetat | | 0,0006 g |
| Natriumsulfit | | 0,25 g |
| Äthylendiamintetraessigsäure | | 0,2 g |
| Parfum | | 0,2 g |
| Enth. Wasser | auf | 100,00 g |

Beispiel 4b

Oxidationsfärbung

20 ml Färbepaste der unten angegebenen Rezeptur für die Farbe "Kupferblond" mit einem Zusatz von 0,0006 % Magnesiumacetat wurde mit 40 ml einer 2 % $H_2O_2$ enthaltenden Entwicklerlösung vermischt, woraus ein Haarfärbemittel mit einem pH-Wert von 6,8 resultierte.

Das Mittel wurde auf ein mittelstarkes Haar mit dem Grundton mittelaschblond aufgetragen und 20 Minuten zur Einwirkung gebracht.

Als Ergebnis wurde eine Umfärbung des Haars in einen dezenten Kupferton erhalten.

Versuche ergaben, daß die Einwirkungszeit des Haarfärbemittels entsprechend der gewünschten resultierenden Farbintensität zwischen 5 und 30 Minuten variiert werden kann.

Rezeptur der Färbepaste "Kupferblond":

| | |
|---|---|
| Cetylstearylalkohol | 10,0 g |
| Kokosfettsäuremonoäthanolamid | 2,0 g |
| Stearinsäuremonoäthanolamid | 2,0 g |
| Stearinsäurediäthanolamid | 1,0 g |
| p-Toluylendiaminsulfat | 0,20 g |
| p-Aminophenol | 0,70 g |
| p-Amino-o-kresol | 0,70 g |
| Monoäthanolamin | 0,2 g |
| Ammoniumchlorid | 0,2 g |
| Natriumlaurylsulfat | 0,3 g |
| Magnesiumacetat | 0,0006 g |
| Natriumsulfit | 0,12 g |
| Äthylendiamintetraessigsäure | 0,2 g |
| Parfum | 0,2 g |
| Enth. Wasser auf | 100,00 g |

Beispiel 5

Oxidationsfärbung zum schonenden, sauren Ausgleich der Haarspitzen nach normaler, alkalischer Ansatzfärbung

20 ml Färbepaste der beim Beispiel 4a angegebenen Rezeptur für die Farbe "Haselnußblond" mit einem Zusatz von 0,0004 % Magnesiumacetat wurde mit 40 ml einer 2 % $H_2O_2$ enthaltenden Entwicklerlösung vermischt, woraus ein Haarfärbemittel mit einem pH-Wert von 6,8 resultierte.

Das Mittel wurde auf ein mittelstarkes Haar, welches haselnußblond gefärbt war und dessen Längen und Spitzen ausgeblichen sind und einen Ton von hellaschblond haben, aufgetragen und 10 Minuten zur Einwirkung gebracht.

Als Ergebnis wurde ein dem Ansatz angeglichener Haselnußblond-Farbton der ausgeblichenen Längen und Spitzen erhalten. Die Einwirkungsdauer des Präparats kann entsprechend den auszugleichenden Farbintensitäten zwischen 5 und 15 Minuten liegen.

Beispiel 6a

Oxidationsfärbung in Verbindung mit der Dauerwellung

Auf mittelstarkem Haar, das einen Grundton von dunkelgoldblond hatte und dessen Haarlängen und -spitzen ausgeblichen waren, wurde eine Dauerwellung durchgeführt.

Die Dauerwelle wurde bis einschließlich der ersten Fixierung auf dem Wickler nach Gebrauchsanweisung verarbeitet.

Zur Nachfixierung und zum gleichzeitigen Färben wurde nach dem Entfernen der Wickler 20 ml Färbepaste der unten angegebenen Rezeptur für die Farbe "Zyklamen" mit einem Zusatz von 0,0004 % Magnesiumacetat mit 40 ml einer Dauerwell-Fixierung mit einem $H_2O_2$-Gehalt von 2 % vermischt.

Das hieraus resultierende Haarfärbemittel mit einem pH-Wert von 6,8 wurde nun mittels einer Auftrageflasche bzw. eines Schwämmchens auf das Haar aufgetragen und 10 Minuten zur Einwirkung gebracht. Als Ergebnis wurde zusätzlich zur Dauerwellung eine Umfärbung des Haars in einen rotvioletten (Zyklamen)-Farbton erhalten. Je nach gewünschter Farbintensität kann die Einwirkungsdauer zwischen 5 und 15 Minuten variiert werden.

Rezeptur der Färbepaste "Zyklamen":

| | |
|---|---|
| Cetylstearylalkohol | 10,0 g |
| Kokosfettsäuremonoäthanolamid | 2,0 g |
| Stearinsäuremonoäthanolamid | 2,0 g |
| Stearinsäurediäthanolamid | 1,0 g |
| p-Toluylendiaminsulfat | 0,5 g |
| p-Amino-o-kresol | 0,4 g |

7

| p-Aminophenol | | 0,1 g |
| Monoäthanolamin | | 0,2 g |
| Ammoniumchlorid | | 0,2 g |
| Natriumlaurylsulfat | | 0,3 g |
| Magnesiumacetat | | 0,0004 g |
| Natriumsulfit | | 0,25 g |
| Äthylendiamintetraessigsäure | | 0,2 g |
| Parfum | | 0,2 g |
| Enth. Wasser | auf | 100,00 g |

Beispiel 6b

Oxidationsfärbung in Verbindung mit der Dauerwellung

Auf mittelstarkem Haar, das einen Grundton von dunkelgoldblond hatte und dessen Haarlängen und -spitzen ausgeblichen waren, wurde eine Dauerwellung durchgeführt.

Die Dauerwelle wurde bis einschließlich der ersten Fixierung auf dem Wickler nach Gebrauchsanweisung verarbeitet.

Zur Nachfixierung und zum gleichzeitigen Färben wurde nach dem Entfernen der Wickler 20 ml Färbepaste der unten angegebenen Rezeptur für die Farbe "Mahagoni" mit einem Zusatz von 0,0004 % Magnesiumacetat mit 40 ml einer Dauerwell-Fixierung mit einem $H_2O_2$-Gehalt von 2 % vermischt.

Das hieraus resultierende Haarfärbemittel mit einem pH-Wert von 6,8 wurde nun mittels einer Auftrageflasche bzw. eines Schwämmchens auf das Haar aufgetragen und 10 Minuten zur Einwirkung gebracht. Als Ergebnis wurde zusätzlich zur Dauerwellung eine Umfärbung des Haars in einen kräftigen Rotton erhalten. Je nach gewünschter Farbintensität kann die Einwirkungsdauer zwischen 5 und 15 Minuten variiert werden.

Rezeptur der Färbepaste "Mahagoni":

| Cetylstearylalkohol | | 10,0 g |
| Kokosfettsäuremonoäthanolamid | | 2,0 g |
| Stearinsäuremonoäthanolamid | | 2,0 g |
| Stearinsäurediäthanolamid | | 1,0 g |
| p-Toluylendiaminsulfat | | 0,4 g |
| p-Aminophenol | | 0,4 g |
| p-Amino-o-kresol | | 0,4 g |
| o-Nitro-p-Phenylendiamin | | 0,4 g |
| Monoäthanolamin | | 0,2 g |
| Ammoniumchlorid | | 0,2 g |
| Natriumlaurylsulfat | | 0,3 g |
| Magnesiumacetat | | 0,0004 g |
| Natriumsulfit | | 0,25 g |
| Äthylendiamintetraessigsäure | | 0,2 g |
| Parfum | | 0,2 g |
| Enth. Wasser | auf | 100,00 g |

Versuche haben gezeigt, daß neben dem in den Beispielen 1a bis 3a verwendeten Kaliumjodid auch die Alkalimetallsalze Kaliumbichromat und Lithiumchlorid und neben dem in den Beispielen 4a bis 6a angegebenen Magnesiumacetat auch die Erdalkalimetallsalze Kalziumchlorid und Bariumnitrat als Zuschlagstoffe zu Haarfärbemitteln die Wirkung haben, auch bei sauer eingestellter und somit das zu behandelnde Haar schonender Rezeptur eine brillante und farbintensive Umfärbung oder Farbauffrischung des behandelten Haars zu ermöglichen.

**Patentansprüche**

1. Mittel für die oxidative Färbung von menschlichen oder tierischen Haaren, welches unmittelbar vor der Anwendung durch Vermischen eines wenigstens einen Oxidationsfarbstoff enthaltenden Färbemittels mit einem Oxidationsmittel hergestellt ist und einen Katalysator enthält, **dadurch gekennzeichnet,** daß das Färbemittel crem- oder gelförmig ist, daß die Mischung aus Färbe- und Oxidationsmittel auf einen pH-Wert zwischen 5,9 und 6,9 eingestellt ist und daß als Katalysator wenigstens ein Salz aus der Gruppe Kaliumjodid, Lithiumchlorid, Kaliumbichromat, Magnesiumacetat, Kalziumchlorid und Bariumnitrat in einer Konzentration zwischen 0,0004 und 0,001 Gew.% bezogen auf das gebrauchsfertige Haarfärbemittel enthalten ist.

2. Verfahren zur Herstellung des Haarfärbemittels nach Anspruch 1, dadurch gekennzeichnet, daß das Alkali- und/oder Erdalkalimetallsalz dem Oxidationsfarbstoff vor der Zugabe des Oxidationsmittels zugemischt wird.

3. Verfahren zur Herstellung des Haarfärbemittels nach Anspruch 1, dadurch gekennzeichnet, daß das Alkali- und/oder Erdalkalimetallsalz bei der Aufbereitung des Haarfärbemittels zugleich mit oder unmittelbar nach der Vermischung des Oxidationsfarbstoffs mit dem Oxidationsmittel zugegeben wird.

4. Verwendung des Haarfärbemittels nach Anspruch 1 zum oxidativen Färben von menschlichen oder tierischen Haaren, welches unmittelbar vor der Anwendung aus wenigstens einem Oxidationsfarbstoff und einem Oxidationsmittel zum gebrauchsfähigen Haarfärbemittel aufbereitet, auf das zu färbende Haar aufgetragen und eine vorgegebene Zeitdauer zur Einwirkung gebracht wird, worauf das Haarfärbemittel ausgewaschen wird.

5. Verwendung des Haarfärbemittels nach Anspruch 1 zum Nachfärben von im Naturzustand dunklerem heller gefärbtem Haar, wobei zunächst auf die dunkleren, nachgewaschenen hautnahen Haarlängen ein an sich bekanntes alkalisch eingestelltes Haarfärbemittel und dann das schwach saure Haarfärbemittel auf die bereits früher gefärbten anschließenden Haarlängen aufgebracht wird, worauf sowohl das alkalische als auch das schwach saure Haarfärbemittel nach hinreichender Einwirkungszeit aus dem behandelten Haar ausgewaschen werden.

6. Verwendung des Haarfärbemittels nach Anspruch 1 beim Wellen und oxidativen Färben von Haaren, wobei auf das gewaschene und auf Wicklern aufgewickelte Haar zunächst ein Dauerwellpräparat für eine vorgegebene Zeitdauer zur Einwirkung gebracht und dann ausgewaschen wird, worauf das aufgewickelte Haar mit einem flüssigen oxidierenden Fixiermittel vorfixiert und dann nach dem Abnehmen der Wickler mittels des schwach sauren Haarfärbemittels nachfixiert wird, indem dieses für die zur hinreichenden Einfärbung der Haare erforderliche Zeitdauer auf dem Haar zur Einwirkung gebracht und anschließend ausgewaschen wird.

## Claims

1. Agent for the oxidative dyeing of human or animal hair, which is prepared immediately before use by mixing with an oxidizing agent a dye containing at least one oxidation dye, and which contains a catalyst, characterized in that the dye is in cream or gel form, that the mixture of dye and oxidant is adjusted to a pH between 5.9 and 6.9, and that at least one salt from the group, potassium iodide, lithium chloride, potassium bichromate magnesium acetate, calcium chloride and barium nitrate, is contained as catalyst in a concentration between 0.0004 and 0.001 wt.-% with respect to the ready-to-use hair dye.

2. Method for the preparation of the hair dye according to claim 1, characterized in that the alkali metal salt and/or alkaline earth metal salt is admixed with the oxidation dye before the oxidant is added.

3. Method for the preparation of the hair dye according to claim 1, characterized in that the alkali metal salt and/or alkaline earth metal salt is added, in the working up of the hair dye, simultaneously with or immediately after the mixing of the oxidation dye with the oxidizing agent.

4. Use of the hair dye according to claim 1 for the oxidative dyeing of human or animal hair, which [hair dye] is worked up to the ready-to-use hair dye immediately before use from at least one oxidation dye and an oxidant, is applied to the hair that is to be dyed, and is allowed to act for a given period of time, whereupon the hair dye is washed out.

5. Use of the hair dye according to claim 1 for the redyeing of lighter-colored hair which is darker in the natural state, wherein first an alkalinely adjusted hair dye known in itself is applied to the darker regrowth hair lengths close to the scalp and then the weakly acid hair dye is applied to the adjoining, previously dyed hair lengths, whereupon both the alkaline and the weakly acid hair dye is washed out of the treated hair after sufficient time of action.

6. Use of the hair dye according to claim 1 in the waving and oxidative dyeing of hair, wherein first a permanent-wave preparation is made to act for a given period of time on the washed hair wound upon curlers and is then washed out, whereupon the wound hair is pre-fixed with a fluid oxidizing fixative and then, after removal of the curlers, is post-fixed by means of the weakly acid hair dye by making the latter to act on the hair for the period of time necessary for the sufficient coloring of the hair and then washing it out.

## Revendications

1. Produit pour la teinture oxydante des cheveux ou des poils, préparé immédiatement avant l'application par mélange d'une teinture contenant au moins un colorant d'oxydation avec un agent oxydant, et qui contient un catalyseur, caractérisé en ce que la teinture est à l'état de crème ou de gel, le mélange de la teinture et de l'agent oxydant est réglé à un pH de 5,9 à 6,9 et le catalyseur consiste en au moins un sel du groupe formé par l'iodure de potassium, le chlorure de lithium, le bichromate de potassium, l'acétate de magnésium, le chlorure de calcium et le nitrate de baryum, qui est contenu à une concentration de 0,0004 à 0,001 % en poids, par rapport à la teinture prête à l'emploi.

2. Procédé de préparation de la teinture selon la revendication 1, caractérisé en ce que le sel de métal alcalin et/ou alcalino-terreux est mélangé avec le colorant d'oxydation avant addition de l'agent oxydant.

3. Procédé de préparation de la teinture selon la revendication 1, caractérisé en ce que le sel de métal alcalin et/ou alcalino-terreux est ajouté à la préparation de la teinture lors du mélange du colorant d'oxydation avec l'agent oxydant ou immédiatement après ce mélange.

4. Utilisation de la teinture selon la revendication 1 pour la teinture oxydante des cheveux ou des poils, dans laquelle on prépare la teinture prête à l'emploi immédiatement avant l'application à partir d'au moins un colorant d'oxydation et d'un agent oxydant, on applique la teinture sur les cheveux ou poils à teindre et on la fait agir pendant une durée déterminée, après quoi on l'élimine par lavage.

5. Utilisation de la teinture selon la revendication 1 pour reteindre des cheveux de couleur sombre à l'état naturel mais teints en une nuance plus claire, dans laquelle on applique d'abord sur les parties des cheveux proches du cuir chevelu, plus sombres, et lavées, une teinture pour cheveux réglée alcaline de type connu en soi puis on applique la teinture pour cheveux légèrement acide sur les parties des cheveux plus éloignées du cuir chevelu, qui ont déjà été teintes antérieurement, après quoi on élimine par lavage la teinture pour cheveux alcaline et la teinture pour cheveux légèrement acide après une durée d'action suffisante sur les cheveux soumis au traitement.

6. Utilisation de la teinture pour cheveux selon la revendication 1 à l'ondulation et à la teinture oxydante des cheveux, dans laquelle, sur les cheveux lavés et sur rouleaux, on fait d'abord agir une préparation pour ondulation permanente pendant une durée déterminée et on l'élimine par lavage, on soumet les cheveux, toujours sur rouleaux, à fixation préalable à l'aide d'un fixateur oxydant liquide puis, après avoir retiré les rouleaux, on soumet à fixation complémentaire à l'aide de la teinture pour cheveux légèrement acide en faisant agir celle-ci sur les cheveux pendant la durée nécessaire pour provoquer une coloration suffisante des cheveux, après quoi on l'élimine par lavage.